(19) Europäisches Patentamt
European Patent Office
Office européen des brevets

(11) Veröffentlichungsnummer: **0 388 665 B1**

(12) **EUROPÄISCHE PATENTSCHRIFT**

(45) Veröffentlichungstag der Patentschrift: **27.04.94**

(51) Int. Cl.5: **C07D 249/08**, C07D 231/16, A01N 43/56, A01N 43/653

(21) Anmeldenummer: **90103758.0**

(22) Anmeldetag: **26.02.90**

Die Akte enthält technische Angaben, die nach dem Eingang der Anmeldung eingereicht wurden und die nicht in dieser Patentschrift enthalten sind.

(54) **Substituierte N-Hydroxypyrazole und N-Hydroxytriazole, Verfahren zu ihrer Herstellung und ihre Verwendung zur Bekämpfung von Schädlingen.**

(30) Priorität: **03.03.89 DE 3906772**
**14.12.89 DE 3941296**

(43) Veröffentlichungstag der Anmeldung:
**26.09.90 Patentblatt 90/39**

(45) Bekanntmachung des Hinweises auf die Patenterteilung:
**27.04.94 Patentblatt 94/17**

(84) Benannte Vertragsstaaten:
**BE CH DE DK ES FR GB IT LI NL**

(56) Entgegenhaltungen:
**EP-A- 0 089 115**
**EP-A- 0 249 977**
**EP-A- 0 289 919**

**CHEMICAL ABSTRACTS Band 102, Nr. 19, 13.May 1985, Seite 591, Spalte 1, Zusammenfassung Nr. 166459f, Columbus, Ohio, US; & JP-A-60 06645**

(73) Patentinhaber: **BASF Aktiengesellschaft**
**Carl-Bosch-Strasse 38**
**D-67063 Ludwigshafen(DE)**

(72) Erfinder: **Neubauer, Hans-Juergen, Dr.**
**Mozartstrasse 6**
**D-4400 Muenster-Hiltrup(DE)**
Erfinder: **Kardorff, Uwe, Dr.**
**D 3,4**
**D-6800 Mannheim 1(DE)**
Erfinder: **Leyendecker, Joachim, Dr.**
**Stahlbuehlring 79**
**D-6802 Ladenburg(DE)**
Erfinder: **Baus, Ulf, Dr.**
**Keltenweg 10**
**D-6915 Dossenheim(DE)**
Erfinder: **Kuenast, Christoph, Dr.**
**Salierstrasse 2**
**D-6701 Otterstadt(DE)**
Erfinder: **Hofmeister, Peter, Dr.**
**Bernard-Humblot-Strasse 12**
**D-6730 Neustadt(DE)**
Erfinder: **Krieg, Wolfgang, Dr.**
**Saarstrasse 17**
**D-6721 Weingarten(DE)**
Erfinder: **Kirstgen, Reinhard, Dr.**
**Erkenbrechtstrasse 23 e**
**D-6730 Neustadt(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 388 665 B1

Erfinder: **Reuther, Wolfgang, Dr.**
**Am Pferchelhang 16**
**D-6900 Heidelberg-Ziegelhausen(DE)**

## Beschreibung

Die vorliegende Erfindung betrifft neue substituierte N-Hydroxypyrazole oder N-Hydroxytriazole der allgemeinen Formel I,

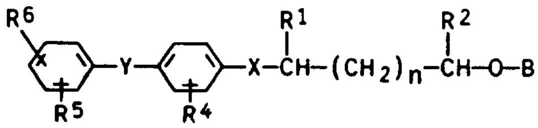

(I),

in der die Substituenten die folgende Bedeutung haben:

| | |
|---|---|
| X, Y | O, S, SO, $SO_2$, $CH_2$, |
| B | einen N-Pyrazol- oder N-1,2,4-Triazolrest der Formeln Ba bzw. Bb |

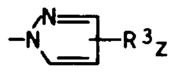

Ba

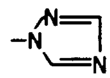

Bb

| | |
|---|---|
| $R^1$, $R^2$ | Wasserstoff, $C_1$-$C_3$-Alkyl, |
| $R^3$ | Wasserstoff, Halogen, $C_1$-$C_3$-Alkyl, |
| z | 1, 2 oder 3, |
| $R^4$, $R^5$, $R^6$ | Wasserstoff, Halogen, $C_1$-$C_4$-Alkyl, $C_1$-$C_4$-Alkoxy, $C_1$-$C_4$-Halogenalkyl, $C_1$-$C_4$-Halogenalkoxy, |
| n | 0, 1, 2. |

Außerdem betrifft die vorliegende Erfindung die Herstellung der Verbindungen I, Schädlingsbekämpfungsmittel, die die Verbindungen I enthalten und ein Verfahren zur Bekämpfung von Schädlingen.

Aus GB-A-2 115 812 sind gewisse 0-substituierte Oxime bekannt. Die insektizide Wirkung der dort beschriebenen Verbindungen ist jedoch unbefriedigend. Auch die in der EP-A-289 919 (DE-A-37 14 709) beschriebenen (p-Phenoxy-phenoxy)-methyl-pyrazole sind unter bestimmten Bedingungen, z.B. niedrigen Aufwandmengen, in ihrer insektiziden Wirkung nicht immer befriedigend.

Der Erfindung lag die Aufgabe zugrunde, neue insektizide Wirkstoffe auf Basis von N-Hydroxypyrazolen oder -triazolen zur Verfügung zu stellen, die in ihrer Wirkung bekannten Verbindungen überlegen sind.

Demgemäß wurde gefunden, daß die eingangs definierten substituierten N-Hydroxypyrazole und -N-Hydroxytriazole der Formel I sich ausgezeichnet zur Bekämpfung von Schädlingen eignen.

Die Herstellung der erfindungsgemäßen substituierten N-Hydroxypyrazole und -triazole I erfolgt durch Umsetzung von Verbindungen der Formel II

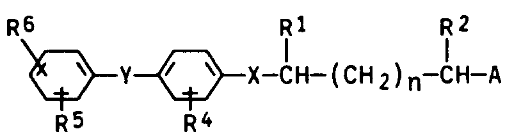

(II),

in der A eine nukleophil verdrängbare Abgangsgruppe bedeutet, mit einem N-Hydroxypyrazol der allgemeinen Formel III

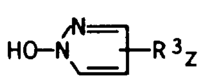

(III),

oder einem N-Hydroxytriazol der Formel IV

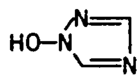

(IV),

in Gegenwart einer Base oder direkt mit dem Alkoholat.

Die Umsetzung kann im Temperaturbereich von (-20) bis 250 °C, vorzugsweise 20 bis 120°C nach folgender Reaktionsgleichung erfolgen:

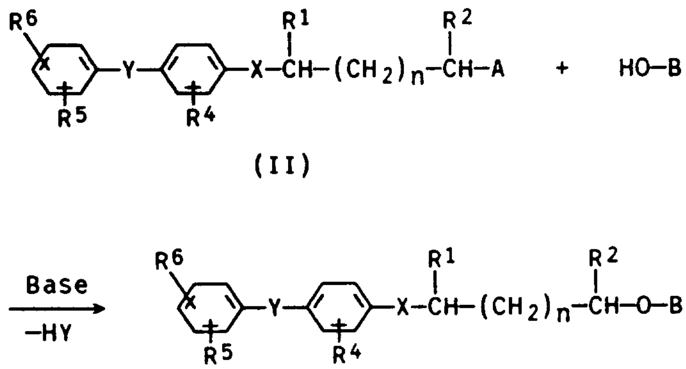


(II)

(I)

In der obigen Reaktionsgleichung bedeutet der Rest A eine an sich übliche Abgangsgruppe, wie beispielsweise einen Sulfonsäurerest oder ein Halogen. Unter den Sulfonsäureresten sind Methansulfonyl, Trifluormethansulfonyl und p-Toluolsulfonyl, unter den Halogenen sind Chlor und Brom bevorzugt; besonders bevorzugt wird Chlor.

Man setzt normalerweise mindestens äquivalente Mengen einer Base, bezogen auf III oder IV, zu II und/oder III oder IV zu, kann aber diese auch im Überschuß oder gegebenenfalls auch als Lösungsmittel verwenden. Als Basen eignen sich beispielsweise Hydroxide von Alkali- und Erdalkalimetallen wie Natriumhydroxid, Kaliumhydroxid und Calciumhydroxid. Alkoholate von Alkali- und Erdalkalimetallen wie Natriummethylat, Natriumethylat, Calciummethanolat oder Kalium-tert.-butylat; Alkali- oder Erdalkalimetallhydride wie Natriumhydrid, Kaliumhydrid oder Calciumhydrid; Alkali- oder Erdalkalicarbonate wie Natriumcarbonat, Kaliumcarbonat oder Calciumcarbonat; aliphatische Amine wie Dimethylamin, Triethylamin oder Diisopropylamin; heterocyclische Amine wie Piperidin, Piperazin oder Pyrrolidin; aromatische Amine wie Pyridin oder Pyrrol und gegebenenfalls auch Alkyllithium-Verbindungen wie n-Butyllithium.

Zur Herstellung der erfindungsgemäßen Verbindung I nach der vorstehend beschriebenen Methode setzt man die Ausgangsstoffe üblicherweise in stöchiometrischem Verhältnis ein. Ein überschuß des einen oder anderen kann in Einzelfällen aber durchaus vorteilhaft sein.

Die Umsetzung wird zweckmäßigerweise in einem Lösungsmittel oder Verdünnungsmittel durchgeführt. Hierzu eignen sich beispielsweise aliphatische Kohlenwasserstoffe wie n-Pentan, n-Hexan, das Hexan-Isomerengemisch und Petrolether; aromatische Kohlenwasserstoffe wie Benzol, Toluol, die Xylole und deren Isomerengemische, Benzin; Alkohole wie Methanol, Ethanol, n-Propanol und Isopropanol; Ether wie Diethylether, Di-n-butylether, Methyl-tert.-butylether, Tetrahydrofuran und Dioxan; Ketone wie Aceton, Methylethylketon und Methylisopropylketon; Nitrile wie Acetonitril und Propionitril; aprotische dipolare Lösungsmittel wie Dimethylformamid, Dimethylsulfoxid oder Pyridin. Auch Gemische dieser Stoffe können als Lösungs- und Verdünnungsmittel verwendet werden.

Die Umsetzungen verlaufen gewöhnlich oberhalb von -20 °C mit ausreichenden Geschwindigkeiten. 120 °C müssen im allgemeinen nicht überschritten werden. Da sie in einigen Fällen unter Wärmeentwicklung verlaufen, kann es von Vorteil sein, Kühlmöglichkeiten vorzusehen.

Die Reaktionsgemische werden in üblicher Weise aufgearbeitet, z. B. durch Versetzen mit Wasser, Trennen der Phasen und Säulenchromatographie. Die neuen Verbindungen der Formel I fallen teilweise in Form farbloser oder schwach bräunlich gefärbter, zäher Öle an, die sich durch längeres Erwärmen unter vermindertem Druck auf mäßig erhöhte Temperatur ("Andestillieren") von den letzten flüchtigen Anteilen befreien und auf diese Weise reinigen lassen. Erhält man die Verbindungen der Formel I in kristalliner Form, so kann ihre Reinigung durch Umkristallisieren erfolgen.

Die benötigten Zwischenprodukte II sind literaturbekannt und können beispielsweise nach den in GB-A-2 115 812 beschriebenen Verfahren gemäß folgendem Reaktionsschema hergestellt werden:

Route 1:

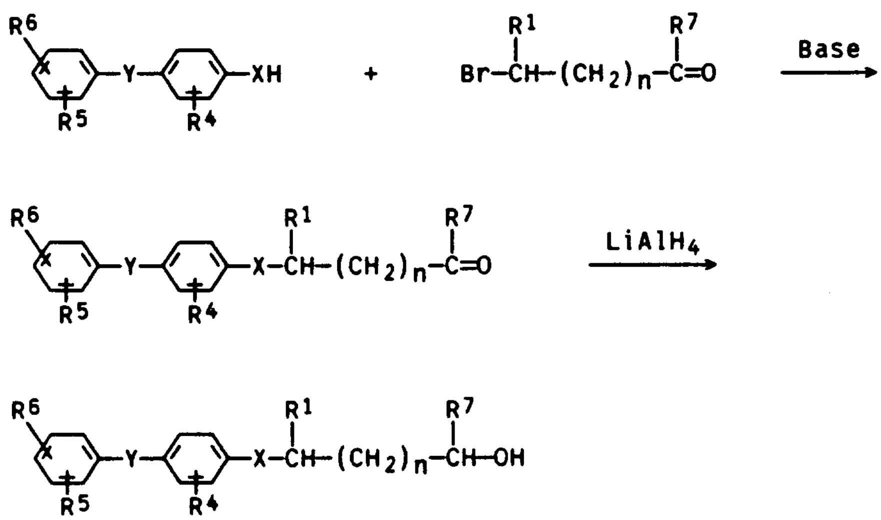


wobei $R^1$, $R^2$, $R^4$, $R^5$, $R^6$, X, Y und n die eingangs definierten Bedeutungen haben und $R^7$ im Fall von $R^2$ = Wasserstoff eine Gruppe $-OR^8$ mit $R^8$ = $C_1$-$C_3$-Alkyl und im Fall von $R^2$ = $C_1$-$C_3$-Alkyl die entsprechende $C_1$-$C_3$-Alkylgruppe darstellt.

Die nach o.g. Schema erhaltene Hydroxyverbindung kann in an sich bekannter Weise in andere nukleophil verdrängbare Abgangsgruppen überführt werden, z.B. durch Umsetzung mit Phosphortribromid.

Stellt A in Ausgangsstoff II ein Halogenatom dar, so kann auch die folgende Route 2 zur Herstellung herangezogen werden:

Route 2

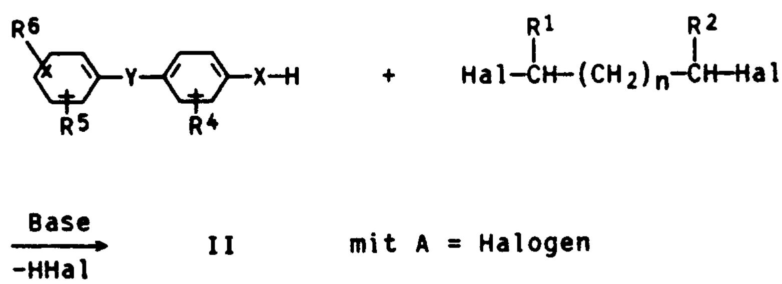


Die Reste $R^1$, $R^2$, $R^4$-$R^6$, X, Y und n haben die voranstehende Bedeutung, Hal steht für Halogen, z.B. Brom oder Chlor.

Die für beide Routen verwendeten Ausgangsverbindungen sind bekannt oder können in an sich bekannter Weise (s. z.B. Angew. Chem. 52, 915 (1938) oder die japanische Patentveröffentlichung Nr. 62033/1980) hergestellt werden.

Zur Darstellung der als Ausgangsverbindungen benötigten N-Hydroxypyrazole der Formel III werden Pyrazole der allgemeinen Formel V zunächst in an sich bekannter Weise mit einem Alkalihydroxid, Alkalihydrid oder Alkalicarbonat in ihre Metallsalze der allgemeinen Formel VI ($Me^+$ bedeutet ein Kation der Alkalimetalle) überführt. Die erhaltenen Metallsalze der Formel VI werden anschließend in einem inerten organischen Lösungsmittel (z.B. Tetrahydrofuran) oder in einem Zweiphasensystem (z.B. Toluol/Wasser) gegebenenfalls in Gegenwart eines Phasentransferkatalysators (z.B. Benzyltriethylammoniumchlorid) mit Dibenzoylperoxid umgesetzt. Die Reaktionstemperatur liegt im allgemeinen zwischen 0 und 60 °C.

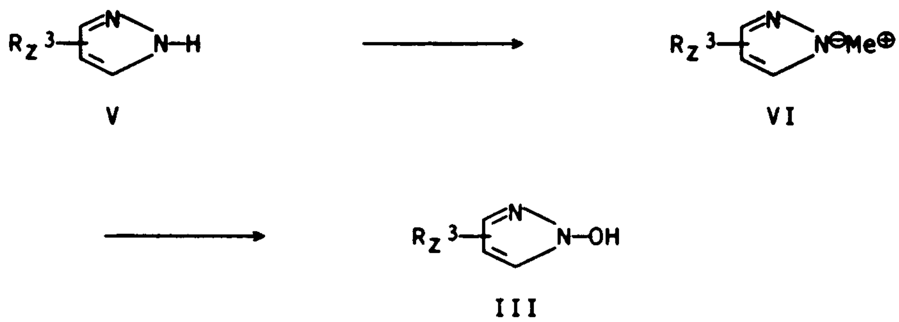

V VI III

Alternativ kann auch so vorgegangen werden, daß man ein Alkalimetallsalz der allgemeinen Formel VI mit einer aliphatischen oder aromatischen Peroxocarbonsäure so umsetzt, daß die Reaktionstemperatur zwischen -5°C und 60°C liegt. Die Umsetzung kann in Wasser als Lösungsmittel oder in einem Zweiphasensystem aus Wasser und einem mit Wasser nur schlecht mischbaren inerten organischen Lösungsmittel (z.B. Toluol), gegebenenfalls in Gegenwart eines geeigneten Phasentransferkatalysators (z.B. Benzyltriethylammoniumchlorid) durchgeführt werden. Die Peroxocarbonsäure kann vor der Reaktion im Reaktionsgemisch aus $H_2O_2$ und einem Carbonsäurehalogenid oder einem Carbonsäureanhydrid hergestellt werden oder In Form eines Alkali- oder Erdalkalisalzes eingesetzt werden.

Die N-Hydroxypyrazole sind z.T. auch bekannt oder lassen sich gemäß dem in US 4 492 689 beschriebenen Verfahren herstellen.

Das N-Hydroxytriazol kann nach dem in der älteren deutschen Anmeldung P 39 00 347.7 beschriebenen Verfahren hergestellt werden. Danach setzt man ein 1-H-1,2,4-Triazol VII mit einer Peroxoverbindung, z.B. Wasserstoffperoxid bei Temperaturen von -20°C bis +150°C um. Gegebenenfalls kann man ein Agenz hinzufügen, welches das 1-H-Triazol zunächst in das Salz VII' überführt. Die Umsetzung wird durch folgendes Reaktionsschema dargestellt, worin Me für ein Metall, z.B. Alkalimetall, steht:

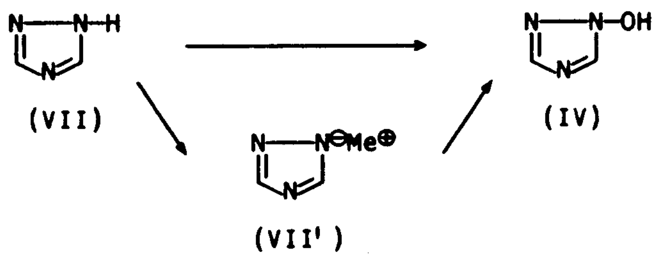

(VII) (IV) (VII')

Für den Fall, daß man das 1-H-1,2,4-Triazol VII mit Peroxogruppen tragenden Verbindungen ohne Zusatz eines salzbildenden Agenzes umsetzt, verfährt man wie folgt:

1 bis 3 Moläquivalente 1-H-1,2,4-Triazol VII werden in einem Lösungsmittel wie Wasser, Wasser/Aceton-Mischung, Tetrahydrofuran, Diglyme, Methylchlorid oder Chloroform mit 1 Moläquivalent einer Peroxocarbonsäure, vorzugsweise m-Chlorperbenzoesäure versetzt. Die Reaktionstemperatur liegt zwischen 0 und 50°C, vorzugsweise bei Raumtemperatur.

Für den Fall, daß man das 1-H-1,2,4-Triazole mit Peroxogruppen tragenden Verbindungen unter Zusatz eines salzbildenden Agenzes umsetzt, verfährt man wie folgt:

a) 1 bis 10 Moläquivalente 1-H-1,2,4-Triazol VII werden in einem inerten Lösungsmittel wie Diglyme, Tetrahydrofuran oder Diethylether mit einer metallorganischen Verbindung einer Alkalimetallsuspension oder einem Hydrid metalliert, anschließend mit 1 Moläquivalent Dibenzoylperoxid versetzt. Man läßt mehrere Tage bei Raumtemperatur rühren.

b) 1 bis 3 Moläquivalente 1-H-1,2,4-Triazol VII werden in Wasser mit einem Hydroxid, einem Carbonat oder einem Hydrocarbonat metalliert, anschließend mit 1 Moläquivalent Peroxocarbonsäure versetzt und über Nacht gerührt. Statt der Peroxocarbonsäure kann man auch das Alkali- oder Erdalkalisalz der Peroxocarbonsäure verwenden und z.B. in fester Form hinzudosieren.

Als salzbildende Agentien eignen sich metallorganische Verbindungen z.B. Metallalkyle wie n-Butyllithium, tert.-Butyllithium und Methyllithium, Metallaryle wie Phenyllithium, Alkalimetallsuspensionen, wie Natrium in Toluol oder Kalium in Toluol, Hydride, z.B. Alkalihydride wie Lithiumhydrid, Natriumhydrid und Kaliumhydrid, Erdalkalihydride wie Calciumhydrid, bevorzugt Natriumhydrid, Hydroxide, z.B. Alkalihydroxide

wie Lithiumhydroxid, Natriumhydroxid und Kaliumhydroxid, Erdalkalihydroxide wie Calciumhydroxid und Magnesiumhydroxid, Carbonate, z.B. Alkalicarbonate wie Lithiumcarbonat, Natriumcarbonat und Kaliumcarbonat, Erdalkalicarbonate wie Calciumcarbonat und Magnesiumcarbonat, Hydrogencarbonate, z.B. Natriumhydrogencarbonat.

Diese Reaktionen können bevorzugt auch in Gegenwart eines Lösungsmittels durchgeführt werden. Bei der Verwendung von metallorganischen Verbindungen oder Hydriden eignen sich Ether wie Diethylether, Methyl-butylether, Tetrahydrofuran und Dioxan, Glykolether wie Diglyme, Triglyme, aliphatische Kohlenwasserstoffe wie Pentan, Hexan, Petrolether, Cyclohexan, aromatische Kohlenwasserstoffe wie Benzol, Toluol und die Xylole oder deren Gemische.

Bei der Verwendung von Hydroxiden, Carbonaten oder Hydrogencarbonaten eignen sich Wasser, Alkohole, wie Methanol, Ethanol, n-Propanol, iso-Propanol und die Butanole, Ketone, wie Aceton und Diethylketon oder deren Gemische, bevorzugt Wasser.

Als Peroxogruppen tragende Verbindungen eignen sich Wasserstoffperoxid oder organische Peroxide, z.B. Dialkylperoxide, Alkylarylperoxide, Diarylperoxide, Diacylperoxide wie Diacetylperoxid, Dipropionylperoxid und Dibenzoylperoxid, bevorzugt Dibenzoylperoxid; Peroxosäuren, z.B. Peroxosulfonsäuren wie p-Toluolperoxosulfonsäure, Toluolperoxosulfonsäure, p-Bromtoluolperoxosulfonsäure und Methylperoxosulfonsäure, bevorzugt p-Toluolperoxosulfonsäure, Peroxocarbonsäuren wie Peroxoessigsäure, Peroxobenzoesäure, m-Chlorperbenzoesäure, Peroxopropionsäure, Peroxobuttersäure, Peroxomaleinsäure, Monoperoxobernsteinsäure und Monoperoxophthalsäure, bevorzugt Monoperoxophthalsäure.

Die Substituenten der Formel I haben im einzelnen folgende Bedeutung:

X, Y — O, S, SO, $SO_2$, $CH_2$, bevorzugt O und S, besonders bevorzugt O,

$R^1$, $R^2$ — Wasserstoff, $C_1$-$C_3$-Alkyl wie Methyl, Ethyl, Propyl, i-Propyl, bevorzugt Wasserstoff und $C_1$-$C_2$-Alkyl, besonders bevorzugt Wasserstoff und Methyl,

$R^3$ — Wasserstoff, Halogen, $C_1$-$C_3$-Alkyl wie für $R^1$ genannt, bevorzugt Wasserstoff, Chlor und Brom, besonders bevorzugt Wasserstoff, Methyl und Chlor,

$R^4$, $R^5$, $R^6$ — Wasserstoff, Halogen, besonders bevorzugt Wasserstoff, Chlor, Brom und Fluor,
$C_1$-$C_4$-Alkyl, bevorzugt $C_1$-$C_3$-Alkyl, besonders bevorzugt Methyl, Ethyl und i-Propyl,
$C_1$-$C_4$-Alkoxy wie Methoxy, Ethoxy, Propoxy, Butoxy, bevorzugt Methoxy und Ethoxy,
$C_1$-$C_4$-Halogenalkyl, z.B. Fluor- oder Chloralkyl, bevorzugt $C_1$-$C_2$-Fluoralkyl, besonders bevorzugt Trifluormethyl,
$C_1$-$C_4$-Halogenalkoxy, bevorzugt $C_1$-$C_3$-Fluoralkoxy, besonders bevorzugt Difluormethoxy, Trifluormethoxy, 1,1,2,2-Tetrafluorethoxy,

n: — die Zahlen 0, 1, 2, bevorzugt 0 und 1, besonders bevorzugt 0,

z: — die Zahlen 1, 2, 3, bevorzugt 1 und 2, besonders bevorzugt sind monosubstituierte (z = 1) oder unsubstituierte Pyrazolreste.

Die erfindungsgemäßen Verbindungen I können ein oder mehrere Asymmetriezentren enthalten. Die vorliegende Erfindung schließt alle möglichen Stereoisomeren wie Diastereomeren und Enantiomeren sowie alle Diastereomeren- und Enantiomerengemische ein.

Die substituierten N-Hydroxypyrazole und N-Hydroxytriazole der allgemeinen Formel I sind geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholita funebrana, Grapholita molesta, Heliothis armigera, Heliothis virescens, Heliothis zea, Hellula undalis, Hibernia defoliaria, Hyphantria cunea, Hyponomeuta malinellus, Keifferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flamea, Pectinophora gossypiella, Peridroma saucia, Phalera bucephala, Phthorimaea operculella, Phyllocnistis citrella, Pieris brassicae, Plathypena scarbra, Plutella xylostella, Pseudoplusia includens, Phyacionia frustrana, Scrobipalpula absoluta, Sitotroga cerelella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Onlema oryzae, Ortiorrhynchus sulcatus, Ortiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossia morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euchistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dyasphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappahis mala, Sappahis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus birittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Zur Klasse der Arachnoidea gehören Spinnentiere (Acarina) beispielsweise Amblyomma americanum, Amglyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobins megnini, Paratetranychus pilosus, Permanyssus gallinae, Phyllocaptrata oleivora, Polyphagotarsonemus latus, Psoroptes ovis, Rhipicephalus appendiculatus, Rhipicephalus evertsi, Saccoptes scabiei, Tetranychus cinnabarinus, Tetranychus kanzawai, Tetranychus pacificus, Tetranychus telarius, Tetranychus urticae.

Zur Klasse der Nematoden zählen beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Hetrodera glycinae, Heterodera schachtii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin-Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Beispiele für Formulierungen sind:

I. 5 Gew.-Teile der Verbindung Nr. 1.1 werden mit 95 Gew.-Teilen feinteiligem Kaolin innig vermischt. Man erhält auf diese Weise ein Stäubemittel, das 3 Gew.-% des Wirkstoffs enthält.

II. 30 Gew.-Teile der Verbindung Nr. 2.1 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

III. 10 Gew.-Teile der Verbindung Nr. 1.2 werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht.

IV. 20 Gew.-Teile der Verbindung Nr. 2.2 werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht.

V. 80 Gew.-Teile der Verbindung Nr. 2.5 werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,1 und 95 Gew.% Wirkstoff, vorzugsweise zwischen 0,5 und 90 Gew.%.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff beträgt unter Freilandbedingungen 0,01 bis 10, vorzugsweise 0,05 bis 2,0 kg/ha.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Herstellungsbeispiele:

A) Substituierte N-Hydroxypyrazole

N-[2-(4-Phenoxyphenoxy)-ethoxy]-pyrazol

(Bsp. Nr. 1.1 der nachfolgenden Tabelle 1)

Zur Suspension von 0,3 g Natriumhydrid in 10 ml Dimethylformamid tropft man unter Kühlung bei 0°C die Lösung aus 1,0 g N-Hydroxypyrazol in 10 ml Dimethylformamid zu. Man läßt noch 1 Stunde nachrühren und versetzt anschließend mit der Lösung von 4,7 g 2-(4-Phenoxyphenoxy)-ethyl-p-toluolsulfonat in 50ml Dimethylformamid. Der Reaktionsansatz wird noch 12 Stunden bei Raumtemperatur gerührt und anschließend auf 250 ml Eiswasser gegossen. Zur Aufarbeitung wird dreimal mit Methyl-tert.-butylether extrahiert, die vereinigten Extrakte mit 1 N Natronlauge und schließlich mit Wasser gewaschen. Nach Trocknen der organischen Phase mit Natriumsulfat, Abziehen des Lösungsmittels im Vakuum und Chromatographie des verbleibenden Rückstandes über Kieselgel mit Toluol als Laufmittel erhält man 2,7 g N-[2-(4-Phenoxyphenoxy)-ethoxy]-pyrazol mit einem Schmelzpunkt von Fp. 83-85°C.

Tabelle 1:

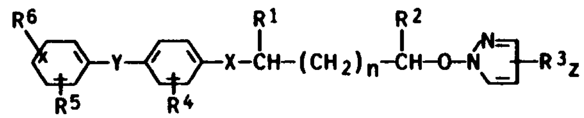

| Nr. | $R^1$ | $R^2$ | $R^3$ | $R^4$ | $R^5$ | $R^6$ | Y | X | n | phys. Daten Fp: (°C), IR: ($cm^{-1}$) |
|---|---|---|---|---|---|---|---|---|---|---|
| 1.1 | H | H | H | H | H | H | O | O | 0 | Fp: 83-85 |
| 1.2 | H | H | 4-Cl | H | H | H | O | O | 0 | Fp: 63-65 |
| 1.3 | H | H | 4-$CH_3$ | H | H | H | O | O | 0 | Fp: 52-56 |
| 1.4 | H | H | H | H | H | H | O | O | 1 | IR: 1588, 1504, 1489, 1474, 1222, 1197 |
| 1.5 | H | H | H | H | 3-F | H | O | O | 0 | Fp: 48-54 |
| 1.6 | H | H | H | H | 4-F | H | O | O | 0 | Fp: 70 |
| 1.7 | H | H | H | H | 2-F | H | O | O | 0 | |
| 1.8 | H | H | H | H | 3-Cl | H | O | O | 0 | Fp: 60-64 |
| 1.9 | H | H | H | H | 3-Br | H | O | O | 0 | Fp: 57-61 |
| 1.10 | H | H | H | H | 3-$CH_3$ | H | O | O | 0 | IR: 1610, 1585, 1503, 1486, 1241, 1209 |
| 1.11 | H | H | H | H | 3-$C_2H_5$ | H | O | O | 0 | IR: 1608, 1583, 1504, 1486, 1236, 1209 |
| 1.12 | H | H | H | H | 3-$OCH_3$ | H | O | O | 0 | IR: 1603, 1590, 1503, 1488, 1210 |
| 1.13 | H | H | H | H | 3-$CF_3$ | H | O | O | 0 | Fp: 61-62 |
| 1.14 | H | H | H | H | 3-F | 5-F | O | O | 0 | |
| 1.15 | H | H | H | H | 3-F | 4-F | O | O | 0 | |
| 1.16 | H | H | H | 3-F | H | H | O | O | 0 | Fp: 68-71 |
| 1.17 | H | $CH_3$ | H | H | H | H | O | O | 0 | IR: 1589, 1505, 1490, 1233 |

Tabelle 1 (Fortsetzung):

| Nr. | $R^1$ | $R^2$ | $R^3$ | $R^4$ | $R^5$ | $R^6$ | Y | X | n | phys. Daten Fp: (°C), IR: ($cm^{-1}$) |
|---|---|---|---|---|---|---|---|---|---|---|
| 1.18 | H | $CH_3$ | H | H | 3-F | H | O | O | 0 | IR: 1614, 1600, 1504, 1485, 1206 |
| 1.19 | H | $CH_3$ | H | H | 4-F | H | O | O | 0 | IR: 1508, 1497, 1235, 1207 |
| 1.20 | H | $CH_3$ | H | H | 2-F | H | O | O | 0 | |
| 1.21 | H | $CH_3$ | H | H | 3-Cl | H | O | O | 0 | IR: 1589, 1504, 1472, 1225 |
| 1.22 | H | $CH_3$ | H | H | 3-Br | H | O | O | 0 | IR: 1584, 1503, 1470, 1222 |
| 1.23 | H | $CH_3$ | H | H | 3-$CH_3$ | H | O | O | 0 | |
| 1.24 | H | $CH_3$ | H | H | 3-$C_2H_5$ | H | O | O | 0 | IR: 1504, 1486, 1234, 1209 |
| 1.25 | H | $CH_3$ | H | H | 3-$OCH_3$ | H | O | O | 0 | |
| 1.26 | H | $CH_3$ | H | H | 3-$CF_3$ | H | O | O | 0 | IR: 1594, 1504, 1492, 1450, 1329 |
| 1.27 | H | $CH_3$ | H | H | 3-F | 5-F | O | O | 0 | IR: 1626, 1601, 1504, 1466, 1208 |
| 1.28 | H | $CH_3$ | H | H | 3-F | 4-F | O | O | 0 | |
| 1.29 | H | $CH_3$ | H | 3-F | H | H | O | O | 0 | |
| 1.30 | H | (S)-$CH_3$ | H | H | H | H | O | O | 0 | |
| 1.31 | H | (R)-$CH_3$ | H | H | H | H | O | O | 0 | |
| 1.32 | $CH_3$ | H | H | H | H | H | O | O | 0 | IR: 1588, 1502, 1489, 1219 |
| 1.33 | $CH_3$ | H | H | H | 3-F | H | O | O | 0 | Fp: 54-55 |
| 1.34 | $CH_3$ | H | H | H | 4-F | H | O | O | 0 | IR: 1505, 1495, 1231, 1206 |
| 1.35 | $CH_3$ | H | H | H | 2-F | H | O | O | 0 | |
| 1.36 | $CH_3$ | H | H | H | 3-Cl | H | O | O | 0 | IR: 1589, 1501, 1472, 1222 |
| 1.37 | $CH_3$ | H | H | H | 3-Br | H | O | O | 0 | |
| 1.38 | $CH_3$ | H | H | H | 3-$CH_3$ | H | O | O | 0 | |
| 1.39 | $CH_3$ | H | H | H | 3-$C_2H_5$ | H | O | O | 0 | |

Tabelle 1 (Fortsetzung):

| Nr. | $R^1$ | $R^2$ | $R^3$ | $R^4$ | $R^5$ | $R^6$ | Y | X | n | phys. Daten Fp: (°C), IR: ($cm^{-1}$) |
|---|---|---|---|---|---|---|---|---|---|---|
| 1.40 | $CH_3$ | H | 4-H | H | 3-$OCH_3$ | H | O | O | 0 | |
| 1.41 | $CH_3$ | H | 4-H | H | 3-$CF_3$ | H | O | O | 0 | |
| 1.42 | $CH_3$ | H | 4-H | H | 3-F | 5-F | O | O | 0 | |
| 1.43 | $CH_3$ | H | 4-H | 3-F | H | H | O | O | 0 | |
| 1.44 | (S)-$CH_3$ | H | 4-H | H | H | H | O | O | 0 | |
| 1.45 | (R)-$CH_3$ | H | 4-H | H | H | H | O | O | 0 | |
| 1.46 | H | $CH_3$ | 4-Cl | H | H | H | O | O | 0 | |
| 1.47 | H | $CH_3$ | 4-Cl | H | 3-F | H | O | O | 0 | |
| 1.48 | H | $CH_3$ | 4-Cl | H | 4-F | H | O | O | 0 | |
| 1.49 | H | $CH_3$ | 4-Cl | H | 2-F | H | O | O | 0 | |
| 1.50 | H | $CH_3$ | 4-Cl | H | 3-Cl | H | O | O | 0 | |
| 1.51 | H | $CH_3$ | 4-Cl | H | 3-Br | H | O | O | 0 | IR: 1584,1502,1470,1222 |
| 1.52 | H | $CH_3$ | 4-Cl | H | 3-$CH_3$ | H | O | O | 0 | |
| 1.53 | H | $CH_3$ | 4-Cl | H | 3-$C_2H_5$ | H | O | O | 0 | |
| 1.54 | H | $CH_3$ | 4-Cl | H | 3-$OCH_3$ | H | O | O | 0 | |
| 1.55 | H | $CH_3$ | 4-Cl | H | 3-$CF_3$ | H | O | O | 0 | |
| 1.56 | H | $CH_3$ | 4-Cl | H | 3-F | 5-F | O | O | 0 | |
| 1.57 | H | $CH_3$ | 4-Cl | H | 3-F | 4-F | O | O | 0 | |
| 1.58 | H | $CH_3$ | 4-Cl | 3-F | H | H | O | O | 0 | |
| 1.59 | $CH_3$ | H | 4-Cl | H | H | H | O | O | 0 | |
| 1.60 | $CH_3$ | H | 4-Cl | H | 3-F | H | O | O | 0 | |
| 1.61 | $CH_3$ | H | 4-Cl | H | 4-F | H | O | O | 0 | |
| 1.62 | $CH_3$ | H | 4-Cl | H | 2-F | H | O | O | 0 | |

Tabelle 1 (Fortsetzung):

| Nr. | $R^1$ | $R^2$ | $R^3$ | $R^4$ | $R^5$ | $R^6$ | Y | X | n | phys. Daten Fp: (°C), IR: ($cm^{-1}$) |
|---|---|---|---|---|---|---|---|---|---|---|
| 1.63 | $CH_3$ | H | 4-Cl | H | 3-Cl | H | O | O | 0 | |
| 1.64 | $CH_3$ | H | 4-Cl | H | 3-Br | H | O | O | 0 | |
| 1.65 | $CH_3$ | H | 4-Cl | H | 3-$CH_3$ | H | O | O | 0 | |
| 1.66 | $CH_3$ | H | 4-Cl | H | 3-$C_2H_5$ | H | O | O | 0 | |
| 1.67 | $CH_3$ | H | 4-Cl | H | 3-$OCH_3$ | H | O | O | 0 | |
| 1.68 | $CH_3$ | H | 4-Cl | H | 3-$CF_3$ | H | O | O | 0 | |
| 1.69 | $CH_3$ | H | 4-Cl | H | 3-F | 5-F | O | O | 0 | |
| 1.70 | $CH_3$ | H | 4-Cl | 3-F | H | H | O | O | 0 | |
| 1.71 | (S)-$CH_3$ | H | 4-Cl | H | H | H | O | O | 0 | |
| 1.72 | (R)-$CH_3$ | H | 4-Cl | H | H | H | O | O | 0 | |
| 1.73 | H | $CH_3$ | 4-$CH_3$ | H | H | H | O | O | 0 | IR: 1589, 1504, 1489, 1223 |
| 1.74 | H | $CH_3$ | 4-$CH_3$ | H | 3-F | H | O | O | 0 | |
| 1.75 | H | $CH_3$ | 4-$CH_3$ | H | 4-F | H | O | O | 0 | IR: 1500, 1222, 1212 |
| 1.76 | H | $CH_3$ | 4-$CH_3$ | H | 2-F | H | O | O | 0 | |
| 1.77 | H | $CH_3$ | 4-$CH_3$ | H | 3-Cl | H | O | O | 0 | |
| 1.78 | H | $CH_3$ | 4-$CH_3$ | H | 3-Br | H | O | O | 0 | |
| 1.79 | H | $CH_3$ | 4-$CH_3$ | H | 3-$CH_3$ | H | O | O | 0 | |
| 1.80 | H | $CH_3$ | 4-$CH_3$ | H | 3-$C_2H_5$ | H | O | O | 0 | IR: 1589, 1503, 1485, 1235, 1208 |
| 1.81 | H | $CH_3$ | 4-$CH_3$ | H | 3-$OCH_3$ | H | O | O | 0 | |
| 1.82 | H | $CH_3$ | 4-$CH_3$ | H | 3-$CF_3$ | H | O | O | 0 | |
| 1.83 | H | $CH_3$ | 4-$CH_3$ | H | 3-F | 5-F | O | O | 0 | |
| 1.84 | H | $CH_3$ | 4-$CH_3$ | 3-F | H | H | O | O | 0 | |
| 1.85 | H | (S)-$CH_3$ | 4-$CH_3$ | H | H | H | O | O | 0 | |

Tabelle 1 (Fortsetzung):

| Nr. | $R^1$ | $R^2$ | $R^3$ | $R^4$ | $R^5$ | $R^6$ | Y | X | n | phys. Daten Fp: (°C), IR: ($cm^{-1}$) |
|---|---|---|---|---|---|---|---|---|---|---|
| 1. 86 | H | (R)-$CH_3$ | 4-$CH_3$ | H | H | H | O | O | 0 | |
| 1. 87 | $CH_3$ | H | 4-$CH_3$ | H | H | H | O | O | 0 | IR: 1588,1502,1489,1219 |
| 1. 88 | $CH_3$ | H | 4-$CH_3$ | H | 3-F | H | O | O | 0 | |
| 1. 89 | $CH_3$ | H | 4-$CH_3$ | H | 4-F | H | O | O | 0 | IR: 1506,1495,1233,1207 |
| 1. 90 | $CH_3$ | H | 4-$CH_3$ | H | 2-F | H | O | O | 0 | |
| 1. 91 | $CH_3$ | H | 4-$CH_3$ | H | 3-Cl | H | O | O | 0 | |
| 1. 92 | $CH_3$ | H | 4-$CH_3$ | H | 3-Br | H | O | O | 0 | |
| 1. 93 | $CH_3$ | H | 4-$CH_3$ | H | 3-$CH_3$ | H | O | O | 0 | |
| 1. 94 | $CH_3$ | H | 4-$CH_3$ | H | 3-$C_2H_5$ | H | O | O | 0 | |
| 1. 95 | $CH_3$ | H | 4-$CH_3$ | H | 3-$OCH_3$ | H | O | O | 0 | |
| 1. 96 | $CH_3$ | H | 4-$CH_3$ | H | 3-$CF_3$ | H | O | O | 0 | |
| 1. 97 | $CH_3$ | H | 4-$CH_3$ | H | 3-F | 5-F | O | O | 0 | |
| 1. 98 | $CH_3$ | H | 4-$CH_3$ | 3-F | H | H | O | O | 0 | |
| 1. 99 | (S)-$CH_3$ | H | 4-$CH_3$ | H | H | H | O | O | 0 | |
| 1.100 | (R)-$CH_3$ | H | 4-$CH_3$ | H | H | H | O | O | 0 | |
| 1.101 | H | H | 4-H | H | H | H | $CH_2$ | O | 0 | |
| 1.102 | H | H | 4-H | H | H | H | S | O | 0 | |
| 1.103 | H | H | 4-H | H | H | H | O | S | 0 | |
| 1.104 | H | H | 4-Cl | H | 3-F | H | O | O | 0 | Fp: 59-61 |
| 1.105 | H | H | 4-Cl | H | 4-F | H | O | O | 0 | |
| 1.106 | H | H | 4-Cl | H | 2-F | H | O | O | 0 | |
| 1.107 | H | H | 4-Cl | H | 3-Cl | H | O | O | 0 | |
| 1.108 | H | H | 4-Cl | H | 3-Br | H | O | O | 0 | IR: 1580,1504,1388 |

Tabelle 1 (Fortsetzung):

| Nr. | $R^1$ | $R^2$ | $R^3$ | $R^4$ | $R^5$ | $R^6$ | Y | X | n | phys. Daten Fp: (°C), IR: ($cm^{-1}$) |
|---|---|---|---|---|---|---|---|---|---|---|
| 1.109 | H | H | 4-Cl | H | 3-$CH_3$ | H | O | O | 0 | Fp: 53-54 |
| 1.110 | H | H | 4-Cl | H | 3-$C_2H_5$ | H | O | O | 0 | IR: 1603,1583,1504,1486, 1236,1209 |
| 1.111 | H | H | 4-Cl | H | 3-$OCH_3$ | H | O | O | 0 | |
| 1.112 | H | H | 4-Cl | H | 3-$CF_3$ | H | O | O | 0 | IR: 1505,1451,1230,1226 |
| 1.113 | H | H | 4-Cl | H | 3-F | 5-F | O | O | 0 | |
| 1.114 | H | H | 4-Cl | 3-F | H | H | O | O | 0 | |
| 1.115 | H | H | 4-$CH_3$ | H | 3-F | H | O | O | 0 | |
| 1.116 | H | H | 4-$CH_3$ | H | 2-F | H | O | O | 0 | |
| 1.117 | H | H | 4-$CH_3$ | H | 4-F | H | O | O | 0 | Fp: 66-70 |
| 1.118 | H | H | 4-$CH_3$ | H | 3-Cl | H | O | O | 0 | IR: 1588,1503,1472,1225 |
| 1.119 | H | H | 4-$CH_3$ | H | 3-Br | H | O | O | 0 | |
| 1.120 | H | H | 4-$CH_3$ | H | 3-$CH_3$ | H | O | O | 0 | |
| 1.121 | H | H | 4-$CH_3$ | H | 3-$C_2H_5$ | H | O | O | 0 | |
| 1.122 | H | H | 4-$CH_3$ | H | 3-$OCH_3$ | H | O | O | 0 | |
| 1.123 | H | H | 4-$CH_3$ | H | 3-$CF_3$ | H | O | O | 0 | |
| 1.124 | H | H | 4-$CH_3$ | H | 3-F | 5-F | O | O | 0 | |
| 1.125 | H | H | 4-$CH_3$ | 3-F | H | H | O | O | 0 | |

B) Substituierte N-Hydroxytriazole

1-(2-[4-(3-Chlor)-phenoxy-phenoxy]ethoxy)-1,2,4-triazol

(Beisp. Nr. 2.19 der nachfolgenden Tabelle 2)

Zu einer Suspension von 0,31 g (12,4 mmol) Natriumhydrid in 3 $cm^3$ Dimethylformamid tropft man unter Eiskühlung eine Lösung aus 0,96 g (11,3 mmol) 1-Hydroxy-1,2,4-triazol in 10 $cm^3$ Dimethylformamid zu. Nach 30-minütigem Rühren wird eine Lösung aus 3,9 g (11,3 mmol) 2-[4-(3-Chlor)-phenoxy-phenoxy]-ethyl-methylsulfonat in 20 $cm^3$ Dimethylformamid bei 60°C zugetropft, anschließend 3 Stunden bei 80°C und weitere 14 h bei Raumtemperatur gerührt. Danach werden flüchtige Bestandteile abgezogen, der Rückstand in ca. 100 $cm^3$ Essigsäureethylester aufgenommen, mit Wasser gewaschen, über Natriumsulfat getrocknet und eingeengt. Es bleibt ein gelbliches Öl zurück.
Ausbeute: 2,9 g (77 % der rechnerisch möglichen Menge).
Infrarotabsorptionen [$cm^{-1}$]:
1502, 1472, 1273, 1222, 1195, 1004, 907, 840, 680.
$^1$H-NMR-Spektrum (300 MHz in $CDCl_3$; δ-Werte in ppm gegen Tetramethylsilan): 4.23 (2H,m,c), 4.71 (2H,m,c), 6.80-7.28 (8H,m), 7.8 (1H,s), 8.11 (1H,s).

Tabelle 2:
Substituierte N-Hydroxytriazole I

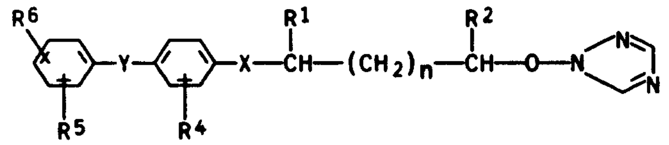
(I),

| Nr. | $R^1$ | $R^2$ | $R^4$ | $R^5$ | $R^6$ | X | Y | n | phys. Daten Fp:(°C), IR($cm^{-1}$) |
|---|---|---|---|---|---|---|---|---|---|
| 2.1 | H | H | H | H | H | O | O | O | Fp:66-67 |
| 2.2 | $CH_3$ | H | H | H | H | O | O | O | 1502,1488,1274,1218, 1198,1005,870,843,681 |
| 2.3 | H | $CH_3$ | H | H | H | O | O | O | |
| 2.4 | H | H | H | 3-F | H | O | O | O | |
| 2.5 | $CH_3$ | H | H | 3-F | H | O | O | O | 1502,1484,1272,1240, 1205,1121,1005,960,681 |
| 2.6 | H | $CH_3$ | H | 3-F | H | O | O | O | 1504,1485,1272,1241, 1206,1121,1004,960,681 |
| 2.7 | H | H | H | 2-F | H | O | O | O | |
| 2.8 | $CH_3$ | H | H | 2-F | H | O | O | O | |
| 2.9 | H | $CH_3$ | H | 2-F | H | O | O | O | |
| 2.10 | H | H | H | 4-F | H | O | O | O | |
| 2.11 | $CH_3$ | H | H | 4-F | H | O | O | O | Fp: 56-57 |
| 2.12 | H | $CH_3$ | H | 4-F | H | O | O | O | |
| 2.13 | H | H | 3-F | H | H | O | O | O | 1507,1486,1253,1245, 1167,976,836,763,680 |
| 2.14 | $CH_3$ | H | 3-F | H | H | O | O | O | |
| 2.15 | H | $CH_3$ | 3-F | H | H | O | O | O | |
| 2.16 | H | H | 3-F | 3-F | H | O | O | O | |
| 2.17 | $CH_3$ | H | 3-F | 3-F | H | O | O | O | |
| 2.18 | H | $CH_3$ | 3-F | 3-F | H | O | O | O | |
| 2.19 | H | H | H | 3-Cl | H | O | O | O | 1502,1472,1273,1222, 1195,1004,907,840,680 |
| 2.20 | $CH_3$ | H | H | 3-Cl | H | O | O | O | 1501,1472,1273,1220, 1195,1005,907,843,681 |
| 2.21 | H | $CH_3$ | H | 3-Cl | H | O | O | O | |
| 2.22 | H | H | H | 2-Cl | H | O | O | O | |
| 2.23 | $CH_3$ | H | H | 2-Cl | H | O | O | O | |
| 2.24 | H | $CH_3$ | H | 2-Cl | H | O | O | O | |
| 2.25 | H | H | H | 4-Cl | H | O | O | O | |
| 2.26 | $CH_3$ | H | H | 4-Cl | H | O | O | O | |

Tabelle 2 (Forts.)

| Nr. | $R^1$ | $R^2$ | $R^4$ | $R^5$ | $R^6$ | X | Y | n | phys. Daten Fp:(°C), IR($cm^{-1}$) |
|---|---|---|---|---|---|---|---|---|---|
| 2.27 | H | $CH_3$ | H | 4-Cl | H | O | O | 0 | |
| 2.28 | H | H | H | 3-Br | H | O | O | 0 | |
| 2.29 | $CH_3$ | H | H | 3-Br | H | O | O | 0 | |
| 2.30 | H | $CH_3$ | H | 3-Br | H | O | O | 0 | |
| 2.31 | H | H | H | 2-Br | H | O | O | 0 | |
| 2.32 | $CH_3$ | H | H | 2-Br | H | O | O | 0 | |
| 2.33 | H | $CH_3$ | H | 2-Br | H | O | O | 0 | |
| 2.34 | H | H | H | 4-Br | H | O | O | 0 | |
| 2.35 | $CH_3$ | H | H | 4-Br | H | O | O | 0 | |
| 2.36 | H | $CH_3$ | H | 4-Br | H | O | O | 0 | |
| 2.37 | H | H | H | 3-$CH_3$ | H | O | O | 0 | |
| 2.38 | $CH_3$ | H | H | 3-$CH_3$ | H | O | O | 0 | |
| 2.39 | H | $CH_3$ | H | 3-$CH_3$ | H | O | O | 0 | |
| 2.40 | H | H | H | 2-$CH_3$ | H | O | O | 0 | |
| 2.41 | $CH_3$ | H | H | 2-$CH_3$ | H | O | O | 0 | |
| 2.42 | H | $CH_3$ | H | 2-$CH_3$ | H | O | O | 0 | |
| 2.43 | H | H | H | 4-$CH_3$ | H | O | O | 0 | |
| 2.44 | $CH_3$ | H | H | 4-$CH_3$ | H | O | O | 0 | |
| 2.45 | H | $CH_3$ | H | 4-$CH_3$ | H | O | O | 0 | |
| 2.46 | H | H | H | 3-$C_2H_5$ | H | O | O | 0 | |
| 2.47 | $CH_3$ | H | H | 3-$C_2H_5$ | H | O | O | 0 | |
| 2.48 | H | $CH_3$ | H | 3-$C_2H_5$ | H | O | O | 0 | 1503, 1485, 1447, 1273, 1234, 1208, 1003, 917 833, 682 |
| 2.49 | H | H | H | 2-$C_2H_5$ | H | O | O | 0 | |
| 2.50 | $CH_3$ | H | H | 2-$C_2H_5$ | H | O | O | 0 | |
| 2.51 | H | $CH_3$ | H | 2-$C_2H_5$ | H | O | O | 0 | |
| 2.52 | H | H | H | 3-i-$C_3H_7$ | H | O | O | 0 | |
| 2.53 | $CH_3$ | H | H | 3-i-$C_3H_7$ | H | O | O | 0 | |
| 2.54 | H | $CH_3$ | H | 3-i-$C_3H_7$ | H | O | O | 0 | |
| 2.55 | H | H | H | 3-i-$C_3H_7$ | H | O | O | 0 | |
| 2.56 | $CH_3$ | H | H | 3-i-$C_3H_7$ | H | O | O | 0 | |
| 2.57 | H | $CH_3$ | H | 3-i-$C_3H_7$ | H | O | O | 0 | |
| 2.58 | H | H | H | 3-i-$C_3H_7$ | H | O | O | 0 | |
| 2.59 | $CH_3$ | H | H | 3-i-$C_3H_7$ | H | O | O | 0 | |
| 2.60 | H | $CH_3$ | H | 3-i-$C_3H_7$ | H | O | O | 0 | |
| 2.61 | H | H | H | 3-$OCH_3$ | H | O | O | 0 | 1503, 1488, 1451, 1274, 1242, 1210, 1138, 682 |

Tabelle 2 (Forts.)

| Nr. | $R^1$ | $R^2$ | $R^4$ | $R^5$ | $R^6$ | X | Y | n | phys. Daten Fp:(°C), IR($cm^{-1}$) |
|---|---|---|---|---|---|---|---|---|---|
| 2.62 | $CH_3$ | H | H | 3-$OCH_3$ | H | O | O | O | |
| 2.63 | H | $CH_3$ | H | 3-$OCH_3$ | H | O | O | O | |
| 2.64 | H | H | H | 2-$OCH_3$ | H | O | O | O | |
| 2.65 | $CH_3$ | H | H | 2-$OCH_3$ | H | O | O | O | |
| 2.66 | H | $CH_3$ | H | 2-$OCH_3$ | H | O | O | O | |
| 2.67 | H | H | H | 4-$OCH_3$ | H | O | O | O | |
| 2.68 | $CH_3$ | H | H | 4-$OCH_3$ | H | O | O | O | |
| 2.69 | H | $CH_3$ | H | 4-$OCH_3$ | H | O | O | O | |
| 2.70 | H | H | H | 3-$OC_2H_5$ | H | O | O | O | |
| 2.71 | $CH_3$ | H | H | 3-$OC_2H_5$ | H | O | O | O | |
| 2.72 | H | $CH_3$ | H | 3-$OC_2H_5$ | H | O | O | O | |
| 2.73 | H | H | H | 2-$OC_2H_5$ | H | O | O | O | |
| 2.74 | $CH_3$ | H | H | 2-$OC_2H_5$ | H | O | O | O | |
| 2.75 | H | $CH_3$ | H | 2-$OC_2H_5$ | H | O | O | O | |
| 2.76 | H | H | H | 4-$OC_2H_5$ | H | O | O | O | |
| 2.77 | $CH_3$ | H | H | 4-$OC_2H_5$ | H | O | O | O | |
| 2.78 | H | $CH_3$ | H | 4-$OC_2H_5$ | H | O | O | O | |
| 2.79 | H | H | H | 3-$CF_3$ | H | O | O | O | |
| 2.80 | $CH_3$ | H | H | 3-$CF_3$ | H | O | O | O | |
| 2.81 | H | $CH_3$ | H | 3-$CF_3$ | H | O | O | O | |
| 2.82 | H | H | H | 2-$CF_3$ | H | O | O | O | |
| 2.83 | $CH_3$ | H | H | 2-$CF_3$ | H | O | O | O | |
| 2.84 | H | $CH_3$ | H | 2-$CF_3$ | H | O | O | O | |
| 2.85 | H | H | H | 4-$CF_3$ | H | O | O | O | |
| 2.86 | $CH_3$ | H | H | 4-$CF_3$ | H | O | O | O | |
| 2.87 | H | $CH_3$ | H | 4-$CF_3$ | H | O | O | O | |
| 2.88 | H | H | H | 3-$OCF_3$ | H | O | O | O | |
| 2.89 | $CH_3$ | H | H | 3-$OCF_3$ | H | O | O | O | |
| 2.90 | H | $CH_3$ | H | 3-$OCF_3$ | H | O | O | O | |
| 2.91 | H | H | H | 2-$OCF_3$ | H | O | O | O | |
| 2.92 | $CH_3$ | H | H | 2-$OCF_3$ | H | O | O | O | |
| 2.93 | H | $CH_3$ | H | 2-$OCF_3$ | H | O | O | O | |
| 2.94 | H | H | H | 4-$OCF_3$ | H | O | O | O | |
| 2.95 | $CH_3$ | H | H | 4-$OCF_3$ | H | O | O | O | |
| 2.96 | H | $CH_3$ | H | 4-$OCF_3$ | H | O | O | O | |
| 2.97 | H | H | H | 3-$OCF_2CF_2H$ | H | O | O | O | |
| 2.98 | $CH_3$ | H | H | 3-$OCF_2CF_2H$ | H | O | O | O | |
| 2.99 | H | $CH_3$ | H | 3-$OCF_2CF_2H$ | H | O | O | O | |

Tabelle 2 (Forts.)

| Nr. | $R^1$ | $R^2$ | $R^4$ | $R^5$ | $R^6$ | X | Y | n | phys. Daten Fp:(°C), IR($cm^{-1}$) |
|---|---|---|---|---|---|---|---|---|---|
| 2.100 | H | H | H | $2-OCF_2CF_2H$ | H | O | O | 0 | |
| 2.101 | $CH_3$ | H | H | $2-OCF_2CF_2H$ | H | O | O | 0 | |
| 2.102 | H | $CH_3$ | H | $2-OCF_2CF_2H$ | H | O | O | 0 | |
| 2.103 | H | H | H | $4-OCF_2CF_2H$ | H | O | O | 0 | |
| 2.104 | $CH_3$ | H | H | $4-OCF_2CF_2H$ | H | O | O | 0 | |
| 2.105 | H | $CH_3$ | H | $4-OCF_2CF_2H$ | H | O | O | 0 | |
| 2.106 | H | H | H | 3-F | 5-F | O | O | 0 | |
| 2.107 | $CH_3$ | H | H | 3-F | 5-F | O | O | 0 | |
| 2.108 | H | $CH_3$ | H | 3-F | 5-F | O | O | 0 | 1505,1466,1244,1208, 1121,1009,995,836 |
| 2.109 | H | H | 3-F | 3-F | 5-F | O | O | 0 | |
| 2.110 | $CH_3$ | H | 3-F | 3-F | 5-F | O | O | 0 | |
| 2.111 | H | $CH_3$ | 3-F | 3-F | 5-F | O | O | 0 | |

C) Herstellung von 1-Hydroxy-1,2,4-triazol
103,5 g (1,5 mol) 1-H-1,2,4-Triazol wurden in 1344 g (12 mol) 50 %igem wäßrigem Kaliumhydroxid gelöst. Unter Eiskühlung wurden 340 g (3 mol) 30 %iges $H_2O_2$, portionsweise 555 g (3,75 mol) Phthalsäureanhydrid zugegeben und 2 Stunden bei Raumtemperatur (20 bis 30 °C) gerührt. Anschließend wurde mit ca. 35 %iger Schwefelsäure auf einen pH-Wert <1,5 angesäuert, der entstandene Niederschlag abgesaugt und das Filtrat durch quantitative HPLC-Messung untersucht. Man erhielt 19 g (15 %) N-Hydroxy-1,2,4-Triazol, das wie üblich aufgearbeitet wurde; Fp.: 132 °C.

Anwendungsbeispiele

In den folgenden Beispielen wurden die erfindungsgemäßen Verbindungen bzw. sie enthaltende Mittel hinsichtlich ihrer Wirkung auf Schädlinge mit der nachstehenden aus GB-A-2 115 812 bekannten Verbindung A verglichen.

A 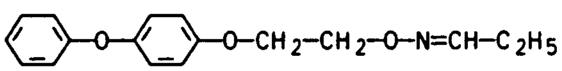

Die Konzentrationen, bei denen die untersuchten Verbindungen eine 100 %ige Mortalität bzw. Hemmung bewirken, sind die jeweiligen Minimal-konzentrationen. Bei jeder Konzentration wurden mindestens 2 Versuche durchgeführt.

Die Reinheit der Wirkstoffe liegt > 95 %. Als Formulierung wurde ein 10 gew.-%iges Emulsionskonzentration gewählt, das durch Emulgieren des Wirkstoffes in einer Mischung aus 70 Gew.-% Cyclohexanon, 20 Gew.-% Nekanil LN® (≙ Lutensol AP6, Netzmittel mit Emulgier- und Dispergierwirkung auf Basis ethoxylierter Alkylphenole) und 10 Gew.-% Emulphor EL® (≙ Emulan EL®, Emulgator auf Basis ethoxylierter Fettalkohole) gewählt. Die in Beispielen angegebenen Konzentrationen wurden durch Verdünnen des formulierten Wirkstoffes mit Wasser erhalten.

Beispiel A

Dysdercus intermedius (Baumwollwanze); ovizide Wirkung

Stecketiketten wurden am oberen Rand mit Klebstreifenstückchen (ca. 0,8 cm) beklebt. 24 Stunden vor Versuchsbeginn wurden die Eier durch Eintauchen in das "Sammelgefäß" an dem Klebstreifenrand befestigt. Die Eier wurden für ca. 5 Sekunden in die wäßrige Wirkstoffaufbereitung getaucht, anschließend ließ man auf Filterpapier abtropfen. Dabei sollen die Eier nicht auf das Papier gelegt werden.

Die Zurechtgeschnittenen Etiketten wurden mit den Klebstreifen nach oben in Gefäße gestellt, die zur Vermeidung von Austrocknung jeweils eine mit Wasser durchfeuchtete, halbe Watterolle enthielten. Nach Abdecken der Gefäße mit Glasplatten wurde gewartet, bis die Larven in einem Kontrollexperiment (ohne Wirkstoff) geschlüpft waren (ca. 8 Tage) und dann bonitiert.

Bei diesem Versuch wurde mit den Verbindungen Nr. 1.1, 1.2, 1.5, 1.104, 2.1, 2.2, 2.6, 2.13 und 2.108 eine bessere Wirkung erzielt als mit der Vergleichsverbindung A, die bei einer Konzentration von 1000 ppm keine Wirkung zeigte.

Beispiel B

Musca domestica (Stubenfliege), Zuchtversuch

Der Versuch wird in 100 ml Kunststoffbechern durchgeführt. Man füllt 25 $cm^3$ (Probierbecher) einer trockenen Futtermischung (1 kg Kleie, 250 g Hefepulver, 35 g Fischmehl) in die Becher, gibt den Wirkstoff mit 25 ml einer Milch-Zuckerlösung (1 l Milch und 42 $cm^3$ Flüssigzucker) zu und mischt mit einem Spatel durch. Danach belegt man jeden Becher mit ca. 30 Larven im ersten Larvenstadium. Die Becher werden mit gelochten Deckeln verschlossen. Der Versuch läuft bis zum Schlüpfen der Fliegen.

Bei diesem Versuch wurde mit den Verbindungen 1.1, 1.5, 1.8, 1.9, 1.13 und 1.104 eine bessere Wirkung erzielt als mit der Vergleichsverbindung A, die bei einer Konzentration von 100 ppm unwirksam war.

Beispiel C

Prodenia litura (ägypt. Baumwollwurm); Zucht

Die Zucht erfolgt in 100 ml Plastikbechern auf ca. 50 ml des Standard-Nährbodens (3,1 l Wasser, 80 g Agar, 137 g Bierhefe, 515 g Maismehl, 130 g Weizenkeime und übliche Zusatzstoffe und Vitamine), dem im flüssigen Zustand der Wirkstoff sorgfältig untergemischt wurde. Je Konzentration wird 1 Becher mit 5 Raupen des vierten Larvenstadiums angesetzt. Die Versuchstemperatur beträgt 25 bis 26 °C. Die Beobachtung erstreckt sich bis zum Schlüpfen der Falter. Die Probe gilt als wirksam, wenn Riesenlarven produziert werden.

Bei diesem Versuch wurde mit den Verbindungen 1.1, 1.8 und 1.9 eine bessere Wirkung erzielt als mit der Vergleichverbindung A.

**Patentansprüche**

**1.** Substituierte N-Hydroxypyrazole oder N-Hydroxytriazole der allgemeinen Formel I

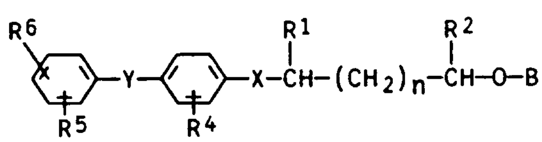

(I),

in der die Substituenten die folgende Bedeutung haben:

X, Y — O, S, SO, $SO_2$, $CH_2$,

B — einen N-Pyrazol- oder N-1,2,4-Triazolrest der Formeln Ba bzw. Bb

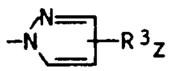 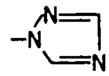

Ba Bb

$R^1$, $R^2$ Wasserstoff, $C_1$-$C_3$-Alkyl,
$R^3$ Wasserstoff, Halogen, $C_1$-$C_3$-Alkyl,
z 1, 2 oder 3,
$R^4$, $R^5$, $R^6$ Wasserstoff, Halogen, $C_1$-$C_4$-Alkyl, $C_1$-$C_4$-Alkoxy, $C_1$-$C_4$-Halogenalkyl, $C_1$-$C_4$-Halogenalkoxy,
n 0, 1, 2.

**2.** Verfahren zur Herstellung der substituierten N-Hydroxypyrazole bzw N-hydroxytriazole der Formel I gemäß Anspruch 1, dadurch gekennzeichnet, daß man eine entsprechend substituierte Verbindung II

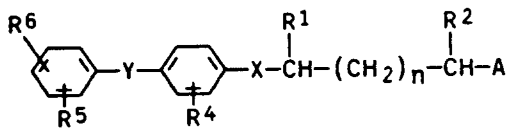 (II),

worin
A eine nukleophil verdrängbare Abgangsgruppe bedeutet,
mit einem N-Hydroxypyrazol der Formel III

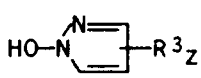 (III),

oder einem N-Hydroxytriazol der Formel IV

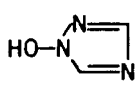 (IV),

in Gegenwart einer Base oder mit dem Alkoholat direkt umsetzt.

**3.** Schädlingsbekämpfungmittel, enthaltend ein substituiertes N-Hydroxypyrazol oder N-Hydroxytriazol gemäß Anspruch 1 neben hierfür üblichen Trägerstoffen.

**4.** Schädlingsbekämpfungmittel nach Anspruch 3, dadurch gekennzeichnet, daß es 0,1 bis 95 Gew.% eines substituierten N-Hydroxypyrazols oder N-Hydroxytriazols gemäß Anspruch 1 enthält.

**5.** Verfahren zur Bekämpfung von Schädlingen aus der Klasse der Insekten, Spinntiere und Nematoden, dadurch gekennzeichnet, daß man die Schädlinge und/oder die von Schädlingen freizuhaltenden Flächen und/oder Räume mit einer für Schädlinge wirksamen Menge eines eines substituierten N-Hydroxypyrazols oder N-Hydroxytriazols der allgemeinen Formel I gemäß Anspruch 1 behandelt.

**6.** Substituierte N-Hydroxypyrazole oder N-Hydroxytriazole der Formel I gemäß Anspruch 1, in der X und Y Sauerstoff bedeuten und n für Null steht.

## Claims

**1.** A substituted N-hydroxypyrazole or N-hydroxytriazole of the general formula I

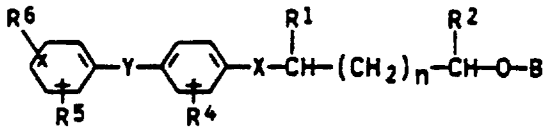

(I),

where
X and Y are O, S, SO, $SO_2$ or $CH_2$,
B is N-pyrazolyl or N-1,2,4-triazolyl of the formulae Ba and Bb respectively

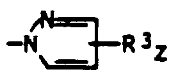

Ba

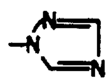

Bb

$R^1$ and $R^2$ are hydrogen or $C_1$-$C_3$-alkyl,
$R^3$ is hydrogen, halogen or $C_1$-$C_3$-alkyl,
z is 1, 2 or 3,
$R^4$, $R^5$ and $R^6$ are hydrogen, halogen, $C_1$-$C_4$-alkyl, $C_1$-$C_4$-alkoxy, $C_1$-$C_4$-haloalkyl or $C_1$-$C_4$-haloalkoxy, and n is 0, 1 or 2

**2.** A process for the preparation of substituted N-hydroxypyrazoles or N-hydroxytriazoles of the formula I as claimed in claim 1, wherein a correspondingly substituted compound II

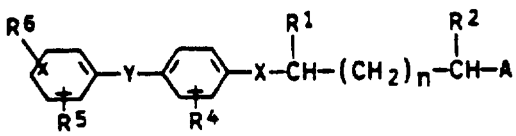

(II),

where
A is a nucleophilically displaceable leaving group, is reacted with an N-hydroxypryazole of the formula III

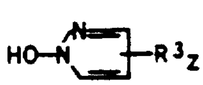

(III),

or an N-hydroxytriazole of the formula IV

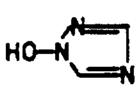

(IV),

in the presence of a base, or directly with the alcoholate

**3.** An agent for controlling pests, containing a substituted N-hydroxypyrazole or N-hydroxytriazole as claimed in claim 1 and conventional carriers therefor.

4. An agent for controlling pests as claimed in claim 3, containing from 0.1 to 95% by weight of a substituted N-hydroxypyrazole or N-hydroxytriazole as claimed in claim 1.

5. A method for controlling pests from the class of insects, arachnida and nematodes, wherein the pests and/or the areas and/or spaces to be kept free from pests are treated with a pesticidally effective amount of a substituted N-hydroxypyrazole or N-hydroxytriazole of the general formula I as claimed in claim 1.

6. A substituted N-hydroxypyrazole or N-hydroxytriazole of the formula I as claimed in claim 1, where X and Y are oxygen and n is 0.

**Revendications**

1. N-hydroxypyrazoles ou N-hydroxytriazoles substitués de la formule générale I

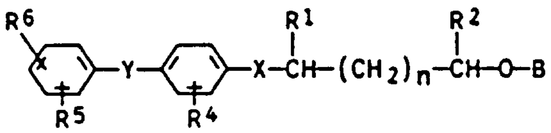

(I),

dans laquelle les substituants ont la signification suivante :

X, Y 0, S, SO, $SO_2$, $CH_2$,
B un reste N-pyrazole ou N-1,2,4-triazole des formules Ba ou Bb

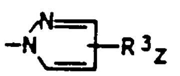

Ba

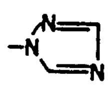

Bb

$R^1$, $R^2$ hydrogène, alkyle en $C_1$-$C_3$,
$R^3$ hydrogène, halogène, alkyle en $C_1$-$C_3$,
Z 1,2 ou 3,
$R^4$, $R^5$, $R^6$ hydrogène, halogène, alkyle en $C_1$-$C_4$, alcoxy en $C_1$-$C_4$, halogénalkyle en $C_1$-$C_4$, halogénalcoxy en $C_1$-$C_4$,
n 0, 1, 2.

2. Procédé de préparation des N-hydroxypyrazoles ou N-hydroxytriazoles substitués de la formule I selon la revendication 1, caractérisé par le fait qu'on fait réagir un composé substitué correspondant II

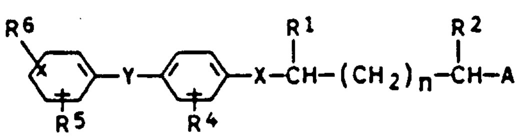

(II),

où
A représente un groupe éliminable déplaçable nucléophile
avec un N-hydroxypyrazole de formule III

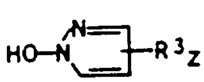

(III),

ou un N-hydroxytriazole de formule IV

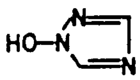 (IV),

en présence d'une base ou directement avec l'alcoolate.

**3.** Pesticide contenant un N-hydroxypyrazole ou N-hydroxytriazole substitué selon la revendication 1 à côté de véhicules usuels.

**4.** Pesticide selon la revendication 3, caractérisé par le fait qu'il contient 0,1 à 95 % en poids d'un N-hydroxypyrazole ou N-hydroxytriazole substitué selon la revendication 1.

**5.** Procédé de lutte contre des pesticides des classes des insectes, arachnides et nématodes, caractérisé par le fait que l'on traite les parasites et/ou les surfaces et/ou volume à libérer des parasites par une quantité à action pesticide d'un N-hydroxypyrazole ou N-hydroxytriazole substitué selon la revendication 1 de formule générale I.

**6.** N-hydroxypyrazole ou N-hydroxytriazole substitué selon la revendication 1 de formule I, dans laquelle X et Y sont mis pour oxygène et n pour zéro.